# EUROPEAN PATENT APPLICATION

(11) **EP 4 835 083 A1**
(43) Date of publication of application: **04.11.2026**
(21) Application number: 24913101.2
(22) Date of filing: 27.12.2024
(51) Int. Cl.: C12N 15/63, C12N 15/12, C12N 15/66, C12Q 1/6811, C12Q 1/6827

(54) **RECOMBINANT VECTOR FOR USE IN PRODUCTION OF TARGET SEQUENCE HAVING POLYMORPHIC NUCLEOTIDE INCORPORATED THEREIN**

(30) Priority: 28.12.2023 JP 2023223318
(71) Applicant: Denka Company Limited, Tokyo 103-8338 (JP)
(72) Inventor: KUBO Hiroshi, Tokyo 103-8338 (JP)
(74) Representative: Müller-Boré & Partner Patentanwälte PartG mbB
(86) International application number: PCT/JP2024/046377
(87) International publication number: WO 2025/143215

(57) **Abstract**

The present invention provides a recombinant vector for preparing a target sequence incorporating a polymorphic nucleotide, having a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences, wherein the two first restriction enzyme recognition sequences are arranged such that cleavage sites for two first restriction enzymes are located on the sides opposite to the second restriction enzyme recognition sequence, and the first restriction enzyme recognition sequence is a Type IIS restriction enzyme recognition sequence, and the second restriction enzyme recognition sequence is a restriction enzyme recognition sequence different from the first restriction enzyme recognition sequence.

## Description

### Technical Field

The present invention broadly relates to a recombinant vector for preparing a target sequence incorporating a polymorphic nucleotide and the like.

### Background Art

As methods of introducing a desired gene mutation into a genome, a method of introducing a target gene into a genome using a viral vector, a method of introducing a target gene into a genome using a transposon system and the like are known. In any of these methods, a construction of a recombination vector having each gene mutation is indispensable. As a method of introducing a mutation into a vector, a PCR-based method is widely used (Non-Patent Literature 1).

### Citation List

### Non-Patent Literature

Non-Patent Literature 1: Karin L Heckman & Larry R Pease. Gene splicing and mutagenesis by PCR-driven overlap extension. NATURE PROTOCOLS. 2007, 2 (4) 924-932.

### Summary of Invention

### Technical Problem

In conventional PCR-based methods, the entire sequence or a partial sequence of a vector into which a mutation has been introduced is synthesized using a polymerase. Therefore, PCR errors may occur, and for the vector after mutation introduction, it is necessary to confirm not only the mutation-introduced site but also the entire sequence or the PCR- amplified sequence, and the incorporation site, which increases time and costs. In addition, primers used in PCR-based methods are required to be of a high purification grade, which further increases time and costs for synthesizing the primers. Therefore, the development of a simpler and less expensive method of introducing a mutation into a vector is required.

### Solution to Problem

The inventors conducted extensive studies in order to address the above problems, and as a result, found a method of producing a recombinant vector having a desired mutation without using conventional PCR-based methods.

Specifically, this application includes the following inventions.
[1] A recombinant vector for preparing a target sequence incorporating a polymorphic nucleotide, having
   a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences,
   wherein the two first restriction enzyme recognition sequences are arranged such that cleavage sites for two first restriction enzymes are located on the sides opposite to the second restriction enzyme recognition sequence, and
   the first restriction enzyme recognition sequence is a Type IIS restriction enzyme recognition sequence, and the second restriction enzyme recognition sequence is a restriction enzyme recognition sequence different from the first restriction enzyme recognition sequence.
[2] The recombinant vector according to [1], wherein the two first restriction enzyme recognition sequences are arranged at least 3 nucleotides apart.
[3] The recombinant vector according to [1] or [2], wherein the polymorphic nucleotide and its 5'-side and 3'-side flanking sequences have 10 to 200 nucleotide pairs.
[4] The recombinant vector according to any one of [1] to [3], which is a transposon vector.
[5] The recombinant vector according to any one of [1] to [4], wherein the Type IIS restriction enzyme is selected from the group consisting of AlwI, AlwXI, Alw26I, Bbsl, Bbvl, Bbvll, Bcefl, Bccl, Bcgl, BciVI, BinI, Bmrl, Bpml, Bsal, BseRl, Bsgl, BsmAl, BsmBI, BspMI, BsrDI, BstF5I, Earl, Eco31I, Eco57I, Esp3I, Esp3I, Faul, Fokl, Gsul, Hgal, HinGUII, Hphl, Ksp632I, Mboll, Mmel, Mn1I, NgoVIII, PaqCl, Plel, Psrl, RleAI, Sapl, SfaNI, Taqll, Tth111II, Acul, Bsml, Bsrl, BsmFIBseMII, BspQl, and BtgZI.
[6] The recombinant vector according to any one of [1] to [5], wherein the target sequence is derived from genomic DNA.
[7] The recombinant vector according to any one of [1] to [6], wherein the recombinant vector further contains a reference sequence.
[8] The recombinant vector according to [7], wherein the reference sequence is an internal standard sequence derived from genomic DNA.
[9] A kit including:
   1) a recombinant vector for preparing a target sequence incorporating a polymorphic nucleotide, having
      a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences,
      wherein the two first restriction enzyme recognition sequences are arranged such that cleavage sites for two first restriction enzymes are located on the sides opposite to the second restriction enzyme recognition sequence, and
      the first restriction enzyme recognition sequence is a Type IIS restriction enzyme recognition sequence, and the second restriction enzyme recognition sequence is a restriction enzyme recognition sequence different from the first restriction enzyme recognition sequence,
   2) a first nucleic acid sequence to be inserted into a restriction enzyme recognition site, and containing a polymorphic nucleotide and its 5'-side and 3'-side flanking sequences, and
   3) a second nucleic acid sequence to be inserted into a restriction enzyme recognition site and containing a complementary strand of the 2) first nucleic acid sequence.
[10] The kit according to [9], which is used to produce a gene as a calibration reference material.
[11] The kit according to [10], wherein the gene as the calibration reference material is genomic DNA.
[12] The kit according to any one of [9] to [11], wherein the recombinant vector further contains an internal standard sequence derived from genomic DNA.
[13] The kit according to any one of [9] to [12], wherein the 2) first nucleic acid sequence and the 3) second nucleic acid sequence form a double strand.
[14] A method of producing a recombinant vector for preparing a target sequence containing a polymorphic nucleotide, including:
   1) a step of cleaving a recombinant vector with a first restriction enzyme, wherein the recombinant vector has a nucleic acid sequence in which a first restriction enzyme recognition sequence, a second restriction enzyme recognition sequence, and a first restriction enzyme recognition sequence are arranged in that order,
      the two first restriction enzyme recognition sequences are arranged such that cleavage sites for two first restriction enzymes are located on the sides opposite to the second restriction enzyme recognition sequence, and
      the first restriction enzyme recognition sequence is a Type IIS restriction enzyme recognition sequence, and the second restriction enzyme recognition sequence is a restriction enzyme recognition sequence different from the first restriction enzyme recognition sequence; and
   2) a step of ligating a double-stranded nucleic acid containing a region composed of a polymorphic nucleotide in a target sequence and its 5'-side and 3'-side flanking sequences together with their complementary sequences.
[15] The method according to [14], further including a step of preparing a double-stranded nucleic acid by annealing.
[16] The method according to [14] or [15], wherein the double-stranded nucleic acid has a sequence complementary to a cleavage sequence of the first restriction enzyme.
[17] The method according to any one of [14] to [16], further including a step of digesting with a second restriction enzyme.

### Advantageous Effect of Invention

According to the present invention, it is possible to prepare a recombinant vector having a desired mutation without using conventional PCR-based methods.

### Brief Description of Drawings

[Figure 1] Figure 1 is a schematic diagram showing the arrangement of a first restriction enzyme recognition sequence and a second restriction enzyme recognition sequence in a recombinant vector when the first restriction enzyme recognition sequence is cleaved at sites 1 to 5 nucleotides apart from the recognition sequence.
[Figure 2] Figure 2 is a schematic diagram showing a case in which a double-stranded nucleic acid is inserted and ligated after the treatment in the first restriction enzyme recognition sequence where the sites 1 to 5 nucleotides apart from the recognition sequence are cleaved.
[Figure 3] Figure 3 shows a schematic diagram of a KRAS-G12A cassette vector.
[Figure 4] Figure 4 shows, in the upper panel, the results of colony direct PCR performed on colonies of *E. coli* transformed using a ligation reaction solution obtained by ligating a KRAS-G12X/G13X cassette vector treated with BsmBI-v2 and an annealed double-stranded DNA oligo, and shows, in the lower panel, the results obtained by treating the PCR product of the colony direct PCR with Notl.
[Figure 5] Figure 5 shows the results of colony direct PCR performed on colonies of *E. coli* transformed using a ligation reaction solution obtained by ligating a KRAS-A59X/Q61X cassette vector treated with BsmBI-v2 and an annealed double-stranded DNA oligo.

### Description of Embodiments

Hereinafter, an embodiment (hereinafter referred to as "the present embodiment") of the present invention will be described, but the scope of the present invention is not limited to the interpretation of the following embodiment.

### (Method of producing recombinant vector for preparing target sequence containing polymorphic nucleotide)

A first embodiment provides a method of producing a recombinant vector for preparing a target sequence containing a polymorphic nucleotide, including 1) a step of cleaving a recombinant vector with a first restriction enzyme, wherein the recombinant vector has a nucleic acid sequence in which a first restriction enzyme recognition sequence, a second restriction enzyme recognition sequence, and a first restriction enzyme recognition sequence are arranged in that order, the two first restriction enzyme recognition sequences are arranged such that cleavage sites for two first restriction enzymes are located on the sides opposite to the second restriction enzyme recognition sequence, and the first restriction enzyme recognition sequence is a Type IIS restriction enzyme recognition sequence, and the second restriction enzyme recognition sequence is a restriction enzyme recognition sequence different from the first restriction enzyme recognition sequence, and 2) a step of ligating a double-stranded nucleic acid containing a region composed of a polymorphic nucleotide in a target sequence and its 5'-side and 3'-side flanking sequences together with their complementary sequences.

As used herein, the term "polymorphic nucleotide" refers to one or more nucleotides that have undergone substitution, deletion or insertion of a nucleotide(s). In addition, "polymorphic nucleotide" may be used as a concept including mutations. The positions of nucleotides that have undergone substitution, deletion or insertion of a nucleotide(s), the number of nucleotides to be substituted or deleted, and the number of nucleotides to be inserted can be appropriately set according to the purpose. The types of mutations are not particularly limited, and examples thereof include missense mutations, nonsense mutations, frameshift mutations, and silent mutations.

As used herein, the term "recombinant vector" refers to a nucleic acid used for recombining a target gene, which carries the target gene to a gene sequence to be recombined. The gene sequence to be recombined is preferably a genome sequence. The recombinant vector is not particularly limited as long as it enables a target gene to be incorporated into the host's gene, particularly the genome, and can achieve stable expression thereof. Examples of types of recombinant vectors include non-viral vectors such as a transposon vector and a plasmid vector, as well as viral vectors. Among these, a transposon vector is preferable. The transposon vector is a vector containing a region called a transposon that contains a gene encoding a transfer enzyme. Among transposon vectors, a Tol2 transposon vector is preferable.

Although not intended to be interpreted as limiting, according to the present embodiment, it is possible to prepare a vector into which a mutation has been introduced in a simple, rapid, and inexpensive manner without using conventional PCR-based methods. Since the recombinant vector prepared according to the present embodiment has no possibility of PCR errors, it is possible to confirm that the desired mutation has been introduced by examining only the sequence around the mutation-introduced site. In addition, while high-purity grade primers used in conventional PCR-based methods require time and cost for synthesis, the present embodiment does not require such primers, which can reduce the time and cost required for vector preparation.

In addition, when a mutation is introduced into genomic DNA using the recombinant vector prepared according to the present embodiment, it is possible to prepare genomic DNA as a calibration reference material in a simple, rapid, and inexpensive manner, which can be used to evaluate the sensitivity of a gene mutation diagnostic agent.

The present embodiment provides a highly useful method of producing a recombinant vector.

### Step of cleaving with first restriction enzyme

The method according to the present embodiment includes a step of cleaving, with a first restriction enzyme, a recombinant vector having a nucleic acid sequence in which a first restriction enzyme recognition sequence, a second restriction enzyme recognition sequence, and a first restriction enzyme recognition sequence are arranged in that order.

As used herein, the first restriction enzyme (which may be one or two) is a Type IIS restriction enzyme. The Type IIS restriction enzyme generally refers to a restriction enzyme that cleaves at a cleavage site separated from its restriction enzyme recognition sequence. Among Type IIS restriction enzymes, a Type IIS restriction enzyme that has all cleavage sites on one side separated from its restriction enzyme recognition sequence is preferable. In addition, the recognition sequence of the Type IIS restriction enzyme need not be a palindromic sequence. Examples of Type IIS restriction enzymes include AlwI, AlwXI, Alw26I, Bbsl, Bbvl, Bbvll, Bcefl, Bccl, Bcgl, BciVI, BinI, Bmrl, Bpml, Bsal, BseRl, Bsgl, BsmAl, BsmBI, BspMI, BsrDI, BstF5I, Earl, Eco31I, Eco57I, Esp3I, Esp3I, Faul, Fokl, Gsul, Hgal, HinGUII, Hphl, Ksp632I, Mboll, Mmel, Mn1I, NgoVIII, PaqCl, Plel, Psrl, RleAI, Sapl, SfaNl, Taqll, Tth111II, Acul, Bsml, Bsrl, BsmFl, BseMII, BspQl, and BtgZl. Among these, Bsml is preferable.

In addition, as used herein, in a nucleic acid sequence in which a first restriction enzyme recognition sequence, a second restriction enzyme recognition sequence, and a first restriction enzyme recognition sequence are arranged in that order, the two first restriction enzyme recognition sequences may be distinguished from each other: the former as a first restriction enzyme recognition sequence A, and the latter as a first restriction enzyme recognition sequence B. In addition, when viewed from either one of single-stranded sequences of the double-stranded sequence of the second restriction enzyme recognition sequence, one located on the 5'-side may be distinguished as a 5'-side first restriction enzyme recognition sequence, and one located on the 3'-side may be distinguished as a 3'-side first restriction enzyme recognition sequence.

The two first restriction enzyme recognition sequences may be restriction enzyme recognition sequences that the same first restriction enzymes recognize or restriction enzyme recognition sequences that different first restriction enzymes recognize.

When the two first restriction enzyme recognition sequences are restriction enzyme recognition sequences that the same first restriction enzymes recognize, the two first restriction enzyme recognition sequences may be the same sequence. When the two first restriction enzyme recognition sequences are restriction enzyme recognition sequences that the same first restriction enzymes recognize, and the first restriction enzyme is a restriction enzyme that recognizes a plurality of nucleic acid sequences, the two first restriction enzyme recognition sequences may be sequences different from each other.

In addition, when the two first restriction enzyme recognition sequences are restriction enzyme recognition sequences that different first restriction enzymes recognize, the two first restriction enzyme recognition sequences may be sequences different from each other. When the two first restriction enzyme recognition sequences are restriction enzyme recognition sequences that different first restriction enzymes recognize, and the different first restriction enzymes recognize the same sequence, the two first restriction enzyme recognition sequences may be the same sequence.

The two first restriction enzyme recognition sequences may be sequences different from each other as described above, but they are preferably the same sequence, and more preferably the same sequence that the same first restriction enzymes recognize.

As used herein, the second restriction enzyme refers to any restriction enzyme different from the first restriction enzyme, and it preferably has a cleavage site within a nucleic acid sequence in which a first restriction enzyme recognition sequence, a second restriction enzyme recognition sequence, and a first restriction enzyme recognition sequence are arranged in that order, and has only one cleavage site in the recombinant vector. In addition, it preferably has no cleavage site in a double-stranded nucleic acid containing a region composed of a polymorphic nucleotide in a target sequence and its 5'-side and 3'-side flanking sequences together with their complementary sequences, which is inserted in step 2). For example, although not particularly limited, Notl is preferable.

In the nucleic acid sequence in which a first restriction enzyme recognition sequence, a second restriction enzyme recognition sequence, and a first restriction enzyme recognition sequence are arranged in that order, the first restriction enzyme recognition sequences are preferably arranged at least 3 nucleotides apart and more preferably arranged at least 6 nucleotides apart.

In one embodiment, the number of nucleotides between the first restriction enzyme recognition sequences is selected from the group consisting of 3 nucleotides, 4 nucleotides, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides and 20 nucleotides.

The nucleic acid sequence in which a first restriction enzyme recognition sequence, a second restriction enzyme recognition sequence, and a first restriction enzyme recognition sequence are arranged in that order may contain a linker sequence having any number of nucleotides.

A first restriction enzyme recognition sequence, a linker, a second restriction enzyme recognition sequence, and a first restriction enzyme recognition sequence may be arranged in that order, a first restriction enzyme recognition sequence, a second restriction enzyme recognition sequence, a linker, and a first restriction enzyme recognition sequence may be arranged in that order, and a first restriction enzyme recognition sequence, a linker, a second restriction enzyme recognition sequence, a linker, and a first restriction enzyme recognition sequence may be arranged in that order.

In addition, a linker, a first restriction enzyme recognition sequence, a linker, a second restriction enzyme recognition sequence, and a first restriction enzyme recognition sequence may be arranged in that order, a linker, a first restriction enzyme recognition sequence, a second restriction enzyme recognition sequence, a linker, and a first restriction enzyme recognition sequence may be arranged in that order, and a linker, a first restriction enzyme recognition sequence, a linker, a second restriction enzyme recognition sequence, a linker, and a first restriction enzyme recognition sequence may be arranged in that order.

In addition, a first restriction enzyme recognition sequence, a linker, a second restriction enzyme recognition sequence, a first restriction enzyme recognition sequence, and a linker may be arranged in that order, a first restriction enzyme recognition sequence, a second restriction enzyme recognition sequence, a linker, a first restriction enzyme recognition sequence, and a linker may be arranged in that order, and a first restriction enzyme recognition sequence, a linker, a second restriction enzyme recognition sequence, a linker, a first restriction enzyme recognition sequence, and a linker may be arranged in that order.

In addition, a linker, a first restriction enzyme recognition sequence, a linker, a second restriction enzyme recognition sequence, a first restriction enzyme recognition sequence, and a linker may be arranged in that order, a linker, a first restriction enzyme recognition sequence, a second restriction enzyme recognition sequence, a linker, a first restriction enzyme recognition sequence, and a linker may be arranged in that order, and a linker, a first restriction enzyme recognition sequence, a linker, a second restriction enzyme recognition sequence, a linker, a first restriction enzyme recognition sequence, and a linker may be arranged in that order. The number of nucleotides constituting the linker sequence is not particularly limited as long as desired effects are obtained.

In one embodiment, the number of nucleotides constituting the linker sequence is selected from the group consisting of 1 nucleotide, 2 nucleotides, 3 nucleotides, 4 nucleotides, 5 nucleotides, 6 nucleotides, 7 nucleotides, 8 nucleotides, 9 nucleotides and 10 nucleotides.

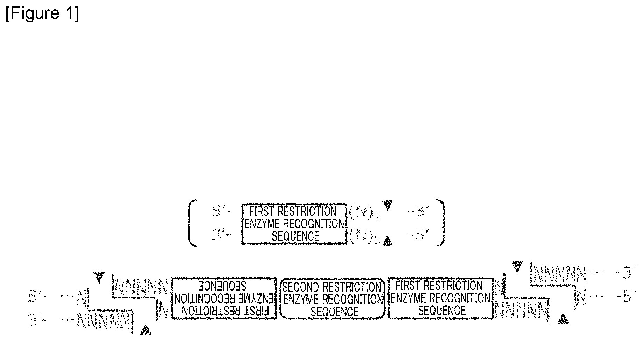

In the nucleic acid sequence in which a first restriction enzyme recognition sequence, a second restriction enzyme recognition sequence, and a first restriction enzyme recognition sequence are arranged in that order, the two first restriction enzyme recognition sequences are arranged such that cleavage sites for two first restriction enzymes are located on the sides opposite to the second restriction enzyme recognition sequence. For example, when the first restriction enzyme recognition sequence is cleaved at sites 1 to 5 nucleotides apart from the recognition sequence, the first restriction enzyme recognition sequence and the second restriction enzyme recognition sequence are arranged such that cleavage sites indicated by underlines (the bond between two underlined nucleotides is cleaved) are represented as: 5'-NNNNNN-(first instance of first restriction enzyme recognition sequence)-(second restriction enzyme recognition sequence)-(second instance of first restriction enzyme recognition sequence)-NNNNNN-3', and in the complementary strand, 3'-NNNNNN- (first instance of first restriction enzyme recognition sequence)-(second restriction enzyme recognition sequence)-(second instance of first restriction enzyme recognition sequence)-NNNNNN-5'. Figure 1 is a schematic diagram showing the arrangement of the first restriction enzyme recognition sequence and the second restriction enzyme recognition sequence in the recombinant vector when the first restriction enzyme recognition sequence is cleaved at sites 1 to 5 nucleotides apart from the recognition sequence. In Figure 1, N represents any nucleotide, and is a part of the target gene sequence in the vicinity of the site where a mutation is to be inserted.

As described above, the recombinant vector containing a nucleic acid sequence in which a first restriction enzyme recognition sequence and a second restriction enzyme recognition sequence are arranged preferably has only two cleavage sites for the first restriction enzyme. That is, the recombinant vector preferably has only two first restriction enzyme recognition sequences.

As described above, a nucleic acid sequence in which a first restriction enzyme recognition sequence and a second restriction enzyme recognition sequence are arranged is cleaved with a first restriction enzyme. Reaction conditions such as the time for reaction with the first restriction enzyme and the treatment temperature can be appropriately determined by those skilled in the art based on the type of the first restriction enzyme.

When a recombinant vector having a nucleic acid sequence in which a first restriction enzyme recognition sequence, a second restriction enzyme recognition sequence, and a first restriction enzyme recognition sequence are arranged in that order is cleaved with a first restriction enzyme, in the recombinant vector, a site having a nucleic acid sequence in which a first restriction enzyme recognition sequence, a second restriction enzyme recognition sequence, and a first restriction enzyme recognition sequence are arranged in that order is excised.

### Step of ligating double-stranded nucleic acid

The method according to the present embodiment includes a step of ligating a double-stranded nucleic acid containing a region composed of a polymorphic nucleotide in a target sequence and its 5'-side and 3'-side flanking sequences together with their complementary sequences.

As used herein, the term "ligation" refers to a reaction in which nucleic acids are linked together via a phosphate diester bond using a ligase. The ligase used in the ligation step and reaction conditions such as the time for reaction with the ligase and the treatment temperature can be appropriately determined by those skilled in the art. Commercially available ligation reagents may be used.

The polymorphic nucleotide in a target sequence and its 5'-side and 3'-side flanking sequences, and their complementary sequences preferably have 10 or more and 200 or less nucleotide pairs.

In one embodiment, the polymorphic nucleotide in a target sequence and its 5'-side and 3'-side flanking sequences, and their complementary sequences have 10 or more and 150 or less nucleotide pairs and preferably have 15 or more and 100 or less nucleotide pairs.

The ligation step is preferably performed after the step of cleaving with the first restriction enzyme.

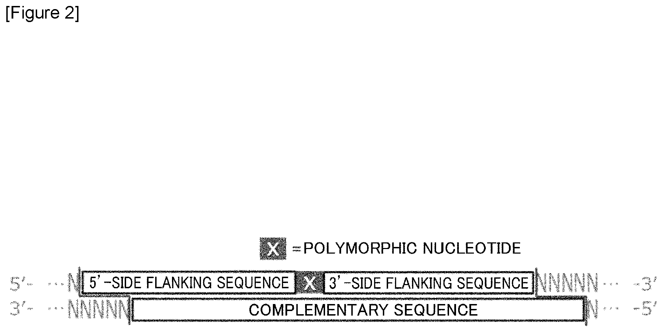

A double-stranded nucleic acid containing a region composed of a polymorphic nucleotide in a target sequence and its 5'-side and 3'-side flanking sequences together with their complementary sequences is inserted into a location where a site having a nucleic acid sequence in which a first restriction enzyme recognition sequence, a second restriction enzyme recognition sequence, and a first restriction enzyme recognition sequence are arranged in that order is excised according to the first restriction enzyme treatment, and ligated. Figure 2 is a schematic diagram showing a case in which the recombinant vector shown as an example in Figure 1 is treated with a first restriction enzyme recognition sequence that cleaves at sites 1 to 5 nucleotides apart from the recognition sequence, and a double-stranded nucleic acid is then inserted and ligated. In Figure 2, N represents any nucleotide, and is a part of the target gene sequence in the vicinity of the site where a mutation is to be inserted, and when the cleavage sequence with the first restriction enzyme has a 5' cohesive end or a 3' cohesive end, N in a region forming a double strand with a part of a 5'-side or 3'-side flanking sequence, and its complementary sequence is a part of a 5'-side or 3'-side flanking sequence and its complementary sequence.

When the cleavage sequence, from which a site having a nucleic acid sequence in which a first restriction enzyme recognition sequence, a second restriction enzyme recognition sequence, and a first restriction enzyme recognition sequence are arranged in that order is excised according to the first restriction enzyme treatment, has a 5' cohesive end or a 3' cohesive end, the double-stranded nucleic acid to be ligated preferably has a 3' cohesive end or a 5' cohesive end such that it forms a complementary strand with the 5' cohesive end or the 3' cohesive end. When the cleavage sequence generated in the recombinant vector according to the first restriction enzyme treatment has only blunt ends, the double-stranded nucleic acid to be ligated may be a nucleic acid having blunt ends.

### Step of annealing double-stranded nucleic acid

The present embodiment may further include a step of annealing the polymorphic nucleotide in a target sequence and its 5'-side and 3'-side flanking sequences with their complementary sequences. The annealing step is preferably performed before the ligation step.

As used herein, annealing refers to a reaction in which a single-stranded nucleic acid and its complementary single-stranded nucleic acid form a double strand. Generally, a double-stranded nucleic acid is formed by treatment at a specific annealing temperature. The annealing temperature is appropriately set by those skilled in the art, and is determined based on, for example, a Tm value specific to the sequence. In the present embodiment, the annealing step is performed by treating a polymorphic nucleotide and its 5'-side and 3'-side flanking sequences, and their complementary sequences at an annealing temperature calculated based on these sequences and the like. The thermal denaturation treatment is performed to form single-stranded nucleic acids, which are then treated at an annealing temperature. The thermal denaturation treatment is performed, for example, at about 95°C. In addition, for example, annealing may be performed by stepwise cooling from the thermal denaturation treatment temperature.

According to the method of the present embodiment, a site having a nucleic acid sequence in which a first restriction enzyme recognition sequence, a second restriction enzyme recognition sequence, and a first restriction enzyme recognition sequence are arranged in that order is excised from the recombinant vector, a double-stranded nucleic acid containing a polymorphic nucleotide is ligated thereto, and thus the recombinant vector containing the polymorphic nucleotide can be prepared.

### Transformation using ligation reaction solution

After the ligation step, it is preferable to perform transformation using a reaction solution after ligation in order to select and purify a recombinant vector containing a polymorphic nucleotide.

Transformation may be performed by any method known to those skilled in the art. Examples of transformation methods include an electroporation method and a heat shock method.

The transformed host is also called a transformant. Selection of transformants, growth of transformants, induction of target protein expression, and collection of hosts expressing target proteins may also be performed by any method known to those skilled in the art.

The transformant can be obtained, for example, by culturing a mixed solution of an expression vector and host competent cells in a medium containing an antibiotic corresponding to a selection marker encoded by the expression vector, and selecting formed colonies. Such a medium varies depending on host cells, and when the host is *E. coli,* an LB medium is used as the medium. The medium used for selecting the transformant is preferably a plate medium. Culture conditions are appropriately adjusted within, for example, a culture temperature of about 30°C to 37°C and a culture time of about 12 hours to overnight.

### Selection of recombinant vector into which double-stranded nucleic acid has been inserted

The fact that the recombinant vector is cleaved with the first restriction enzyme and the double-stranded nucleic acid is inserted can be confirmed by subjecting the formed colonies to a PCR reaction and qualitatively analyzing them. As primers used in the PCR reaction, for example, primers that amplify a region including a double-stranded nucleic acid insertion site can be used. By qualitatively analyzing an amplification product (PCR product) obtained by the PCR reaction to determine whether a PCR amplicon with a desired length has been generated, it can be confirmed whether the double-stranded nucleic acid has been inserted. Qualitative analysis of the PCR product can be performed, for example, by electrophoresis which performs separation based on the molecular weight.

In the PCR reaction, for example, it is preferable to directly use the formed colonies as templates (colony direct PCR), but nucleic acids extracted from the colonies may also be used as templates.

### Selection of recombinant vector into which double-stranded nucleic acid containing polymorphic nucleotide has been inserted

The recombinant vector into which a double-stranded nucleic acid has been inserted also includes those in which, after the recombinant vector is cleaved with the first restriction enzyme, instead of a double-stranded nucleic acid containing a polymorphic nucleotide, a double-stranded nucleic acid containing a nucleic acid sequence in which a first restriction enzyme recognition sequence, a second restriction enzyme recognition sequence, and a first restriction enzyme recognition sequence are arranged in that order, which is excised, is inserted, and self-ligated. In addition, it also includes those that are not cleaved at all with the first restriction enzyme and those that are cleaved at only one site with the first restriction enzyme.

Therefore, when the PCR product in which insertion of the double-stranded nucleic acid has been confirmed is treated with the second restriction enzyme, it is possible to confirm whether the double-stranded nucleic acid containing a polymorphic nucleotide has been inserted into the recombinant vector.

Instead of a double-stranded nucleic acid containing a polymorphic nucleotide, a double-stranded nucleic acid containing a nucleic acid sequence in which a first restriction enzyme recognition sequence, a second restriction enzyme recognition sequence, and a first restriction enzyme recognition sequence are arranged in that order, which is excised with the first restriction enzyme, is inserted and the PCR product derived from the self-ligated recombinant vector is cleaved with the second restriction enzyme. On the other hand, the PCR product derived from the recombinant vector into which a double-stranded nucleic acid containing a polymorphic nucleotide has been inserted is not cleaved with the second restriction enzyme.

After treatment with the second restriction enzyme, qualitative analysis is performed to confirm whether cleavage has occurred with the second restriction enzyme, and thus it is possible to confirm whether the double-stranded nucleic acid containing a polymorphic nucleotide has been inserted into the recombinant vector.

The PCR product in which insertion of the double-stranded nucleic acid containing a polymorphic nucleotide has been confirmed and which has not been cleaved with the second restriction enzyme is preferably subjected to sequence analysis by amplifying the vicinity of the region where the double-stranded nucleic acid containing a polymorphic nucleotide has been inserted, thereby finally confirming that the double-stranded nucleic acid containing a polymorphic nucleotide has been inserted.

Colonies in which insertion of the double-stranded nucleic acid containing a polymorphic nucleotide has been confirmed are grown by culturing, and the target recombinant vector containing a polymorphic nucleotide is purified from the culture product. The target recombinant vector obtained by purification is preferably subjected to sequence analysis by amplifying the vicinity of the region where the double-stranded nucleic acid containing a polymorphic nucleotide has been inserted, thereby finally confirming that the target recombinant vector containing a polymorphic nucleotide has been produced.

When a recombinant vector containing a polymorphic nucleotide is prepared using a conventional PCR method, PCR errors may occur, and thus it is necessary to finally perform full-sequence analysis of the vector to confirm whether the target recombinant vector has been produced. However, in the method of the present embodiment, since a region containing a polymorphic nucleotide is inserted by ligation without using a PCR method, it is possible to confirm whether the target recombinant vector has been produced by sequence only the vicinity of the insertion region.

According to the method of the present embodiment, a recombinant vector containing a polymorphic nucleotide is produced, the recombinant vector can be used to incorporate a polymorphic nucleotide into a nucleic acid, and a target sequence containing the polymorphic nucleotide can be prepared. Therefore, the present embodiment provides a method of producing a recombinant vector for preparing a target sequence containing a polymorphic nucleotide. In the present embodiment, the method of producing a recombinant vector for preparing a target sequence containing a polymorphic nucleotide may be a method of producing a recombinant vector for introducing a target sequence containing a polymorphic nucleotide into a nucleic acid.

The nucleic acid into which a polymorphic nucleotide is incorporated using the recombinant vector produced by the method of the present embodiment is any nucleic acid such as DNA, genomic DNA, plasmid DNA, cDNA, and RNA. Among these, genomic DNA is preferable. When the nucleic acid into which a polymorphic nucleotide is incorporated using the recombinant vector is genomic DNA, the target sequence in the present embodiment is preferably derived from the genomic DNA into which the polymorphic nucleotide is incorporated. That is, the sequence other than the polymorphic nucleotide is preferably derived from the sequence of the genomic DNA into which the polymorphic nucleotide is incorporated.

As used herein, the term "genomic DNA" refers to DNA that constitutes the entire set of genetic information (genome) of cells.

When the recombinant vector produced by the method of the present embodiment is a viral vector, a polymorphic nucleotide is incorporated into a nucleic acid by infecting a host with the viral vector, when the recombinant vector is a non-viral vector, a polymorphic nucleotide is incorporated into a nucleic acid by introducing the recombinant vector into a host by transformation, and when the recombinant vector is a transposon vector, a polymorphic nucleotide is incorporated into a nucleic acid using a transposon system.

The nucleic acid into which a polymorphic nucleotide is incorporated can be used as a calibration reference material.

As used herein, the term "calibration reference material" refers to a material that serves as a reference for evaluating an analysis method or a diagnostic agent or calibrating a device. For example, in order to evaluate the sensitivity of a companion diagnostic agent such as a gene mutation diagnostic agent, a gene having a mutation is used as a calibration reference material, and the mutation can be introduced using a recombinant vector containing the mutation.

Therefore, the method of the present embodiment may be a method of producing a recombinant vector for producing a gene as a calibration reference material. According to the method of the present embodiment, a recombinant vector containing a mutation is produced, and a gene having a mutation as a calibration reference material is produced using the recombinant vector containing the mutation.

The gene used as the calibration reference material is preferably genomic DNA.

When the sensitivity of the gene mutation diagnostic agent is evaluated using the calibration reference material, the sensitivity is evaluated based on the VAF (variant allele frequency, mutant gene frequency) defined for the calibration reference material. Therefore, it is necessary that a VAF be defined for each calibration reference material. In addition, for each mutant gene targeted by the diagnostic agent, a calibration reference material in which the VAF of the mutant gene is defined is required.

For example, when the sensitivity is evaluated using a calibration reference material in which a VAF of 1% is defined, if a mutation can be detected by examining the calibration reference material in which the VAF is defined with a gene mutation diagnostic agent whose sensitivity is to be evaluated, the sensitivity (VAF) of the gene mutation diagnostic agent can be evaluated as 1% or more.

As a method of determining the VAF of a calibration reference material, for example, a method using the copy number of a mutant gene in the calibration reference material measured by a validated droplet digital PCR is known. When the copy number of the mutant gene is used, a validated droplet digital PCR reagent is required for each mutant gene.

When the gene as a calibration reference material is genomic DNA, in order to easily determine the VAF of the calibration reference material, the recombinant vector produced by the method of the present embodiment preferably contains a reference sequence derived from the genomic DNA as the calibration reference material. The reference sequence is preferably an internal standard sequence derived from the genomic DNA as the calibration reference material, and the reference sequence such as the internal standard sequence is preferably incorporated into the calibration reference material together with the polymorphic nucleotide using the recombinant vector.

As used herein, the term "internal standard sequence" refers to any gene sequence derived from genomic DNA into which a polymorphic nucleotide is incorporated using a recombinant vector, which is different from the target sequence in the genomic DNA, but is preferably an internal standard sequence for which a validated droplet digital PCR reagent is commercially available. In addition, a gene sequence in a region near a centromere, where transposon transition or viral gene insertion is unlikely to occur, is preferable. The target sequence and the internal standard sequence may be sequences derived from different gene loci or may be sequences derived from different chromosomes. There is no need to select, as the internal standard sequence, a sequence of a housekeeping gene or the like, which is constitutively expressed in cells and has small expression variation.

Examples of internal standard sequences include the PTEN gene, the AGO1 gene, the TMEM11 gene, and the ERBB2 gene.

When a reference sequence such as an internal standard sequence is incorporated, together with a polymorphic nucleotide, into a calibration reference material, without using the copy number of the mutant gene, it is possible to accurately define the VAF of the calibration reference material using the copy number of the reference sequence such as an internal standard sequence. That is, regardless of which mutant gene the calibration reference material has, the VAF of the calibration reference material can be determined using the copy number of the reference sequence such as an internal standard sequence. When the recombinant vector contains a reference sequence such as an internal standard sequence derived from the calibration reference material, a validated droplet digital PCR reagent for an internal standard gene can be commonly used for calibration reference materials containing respective mutant genes, and thus the VAFs of a plurality of calibration reference materials can be easily determined. Therefore, the method of the present embodiment may be a method of producing a recombinant vector for easily determining the VAF of the calibration reference material. A method of calculating the VAF using an internal standard gene when the calibration reference material is genomic DNA will be described below in detail.

### Method of calculating VAF of genomic DNA as calibration reference material

The internal standard sequence introduced using the recombinant vector together with the target sequence containing a polymorphic nucleotide is referred to herein as a first internal standard sequence.

Since the target sequence and the first internal standard sequence are derived from genomic DNA, the genomic DNA into which the target sequence containing a polymorphic nucleotide and the first internal standard sequence are introduced is genomic DNA containing a non-mutated wild-type target sequence and the first internal standard sequence.

Thus, when one copy of the target sequence containing a polymorphic nucleotide and one copy of the first internal standard sequence are introduced into genomic DNA containing one copy of the wild-type target sequence and one copy of the first internal standard sequence, the resulting genomic DNA contains one copy of the wild-type target sequence, one copy of the target sequence containing a polymorphic nucleotide, and two copies of the first internal standard sequence.

Therefore, in the genomic DNA as the calibration reference material produced by the method of the present embodiment, the copy number of the target sequence containing a polymorphic nucleotide is equal to the copy number of the first internal standard sequence, which is increased by being introduced together with the target sequence containing a polymorphic nucleotide. The increase in the copy number of the first internal standard sequence can be calculated by subtracting the copy number of the first internal standard sequence in the genomic DNA before gene introduction from the copy number of the first internal standard sequence in the genomic DNA after gene introduction (Formula 1). [Formula 1] 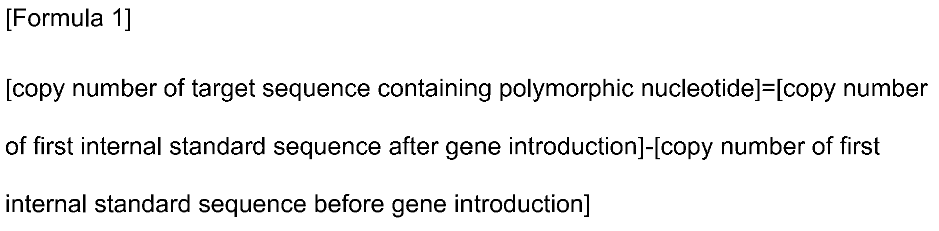 [copy number of target sequence containing polymorphic nucleotide]=[copy number of first internal standard sequence after gene introduction]-[copy number of first internal standard sequence before gene introduction]

In addition, here, any internal standard sequence that is inherent to genomic DNA and different from the first internal standard sequence is referred to as a second internal standard sequence. The first internal standard sequence and the second internal standard sequence may be sequences derived from different gene loci or may be sequences derived from different chromosomes.

When the genomic DNA is derived from a cell in which the chromosome number is diploid, the copy number of the first internal standard sequence and the copy number of the second internal standard sequence are the same in the genomic DNA before the target sequence containing a polymorphic nucleotide and the internal standard sequence are introduced. Even after the target sequence containing a polymorphic nucleotide and the internal standard sequence are introduced, the copy number of the second internal standard sequence does not change (Formula 2). [Formula 2] 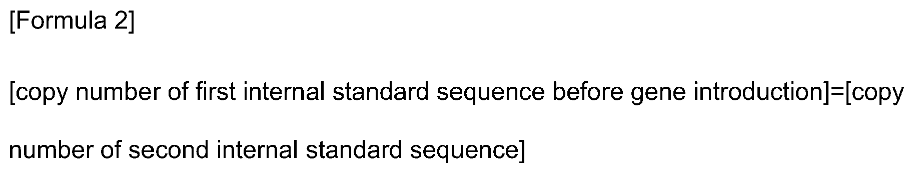 [copy number of first internal standard sequence before gene introduction]=[copy number of second internal standard sequence]

Therefore, from [Formula 1] and [Formula 2], the copy number of the target sequence containing a polymorphic nucleotide in the genomic DNA as the calibration reference material produced by the method of the present embodiment can be calculated by subtracting the copy number of the second internal standard sequence from the copy number of the first internal standard sequence in the genomic DNA after the target sequence containing a polymorphic nucleotide and the first internal standard sequence are introduced (Formula 3). [Formula 3] 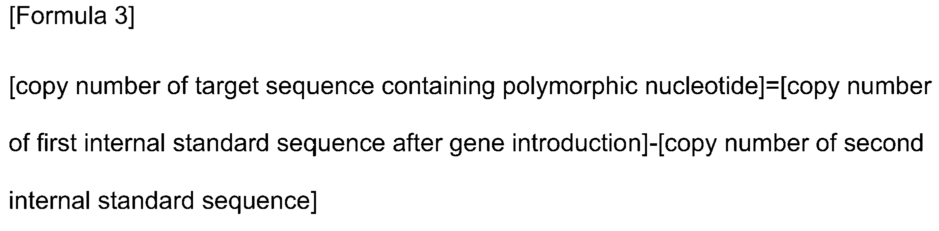 [copy number of target sequence containing polymorphic nucleotide]=[copy number of first internal standard sequence after gene introduction]-[copy number of second internal standard sequence]

In addition, when the genomic DNA is derived from a cell in which the chromosome number is diploid, the copy number of the wild-type target sequence and the copy number of the second internal standard sequence are the same, and this relationship does not change even after the target sequence containing a polymorphic nucleotide and the internal standard sequence are introduced (Formula 4). [Formula 4] 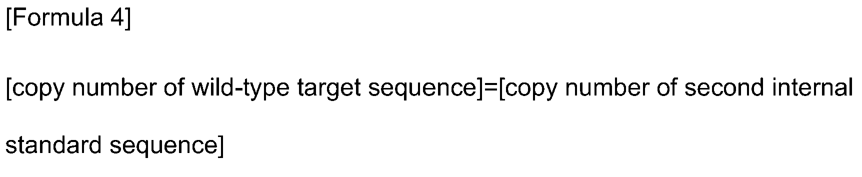 [copy number of wild-type target sequence]=[copy number of second internal standard sequence]

Furthermore, the VAF is generally calculated using the copy number of the target sequence containing a polymorphic nucleotide according to the calculation formula [Formula 5]. [Formula 5] 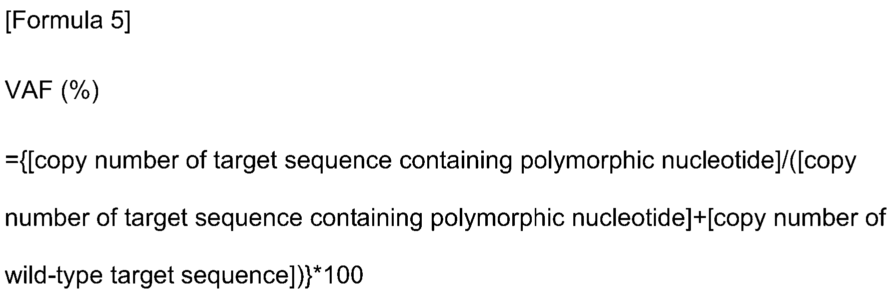 VAF (%) ={[copy number of target sequence containing polymorphic nucleotide]/([copy number of target sequence containing polymorphic nucleotide]+[copy number of wild-type target sequence])}*100

Therefore, from [Formula 3] to [Formula 5], the VAF of the genomic DNA as the calibration reference material can be calculated using the copy number of the first internal standard sequence and the copy number of the second internal standard sequence (Formula 6). [Formula 6] $\begin{matrix}VAF \left(%\right) \\ = \left\{\left(\left[copy number of first internal standard sequence after gene introduction\right]-\left[\begin{matrix}copy \\ number of second internal standard sequence\end{matrix}\right]\right)/\left(\left[\begin{matrix}copy number of first internal \\ standard sequence after gene introduction\end{matrix}\right]-\left[\begin{matrix}copy number of second internal \\ standard sequence\end{matrix}\right]+\left[copy number of second internal standard sequence\right]\right)\right\} * 100 \\ = \left\{\left(\left[copy number of first internal standard sequence after gene introduction\right]-\left[\begin{matrix}copy \\ number of second internal standard sequence\end{matrix}\right]\right)/\left[\begin{matrix}copy number of first internal \\ standard sequence after gene introduction\end{matrix}\right]\right\} * 100\end{matrix}$

The genomic DNA is preferably derived from a cell in which the chromosome number is diploid. However, when the chromosome number is not diploid, the VAF can be calculated in the same manner as described above by selecting the first internal standard sequence and the second internal standard sequence such that the first internal standard sequence, the second internal standard sequence, and the target sequence each have the same number of chromosomes.

Therefore, by incorporating the internal standard sequence into the calibration reference material together with the target sequence containing a polymorphic nucleotide using a recombinant vector, without using the copy number of the mutant gene, the VAF of the calibration reference material can be calculated using the copy numbers of two types of internal standard genes. That is, the VAF of the calibration reference material can be accurately calculated without preparing a validated droplet digital PCR reagent for each mutant gene. Since validated droplet digital PCR reagents for two types of internal standard genes can be commonly used for the calibration reference materials containing respective mutant genes, the VAFs of a plurality of calibration reference materials can be easily calculated.

The method of producing a recombinant vector for preparing a target sequence containing a polymorphic nucleotide according to the first embodiment also applies to second and third embodiments described below.

### (Recombinant vector for preparing target sequence incorporating polymorphic nucleotide)

The second embodiment provides a recombinant vector for preparing a target sequence incorporating a polymorphic nucleotide, having a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences, wherein the two first restriction enzyme recognition sequences are arranged such that cleavage sites for two first restriction enzymes are located on the sides opposite to the second restriction enzyme recognition sequence, and the first restriction enzyme recognition sequence is a Type IIS restriction enzyme recognition sequence, and the second restriction enzyme recognition sequence is a restriction enzyme recognition sequence different from the first restriction enzyme recognition sequence.

The recombinant vector having a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences is a recombinant vector having a nucleic acid sequence in which a polymorphic nucleotide in a target sequence and its 5'-side and 3'-side flanking sequences are replaced with a nucleic acid sequence in which a first restriction enzyme recognition sequence, a second restriction enzyme recognition sequence, and a first restriction enzyme recognition sequence are arranged in that order. That is, it means a recombinant vector in which a polymorphic nucleotide in a target sequence and its 5'-side and 3'-side flanking sequences are excised from a recombinant vector containing a target sequence, and a nucleic acid sequence in which a first restriction enzyme recognition sequence, a second restriction enzyme recognition sequence, and a first restriction enzyme recognition sequence are arranged in that order is inserted into the excised region. The polymorphic nucleotide and its 5'-side and 3'-side flanking sequences may have the same length as or a different length from the nucleic acid sequence in which a first restriction enzyme recognition sequence, a second restriction enzyme recognition sequence, and a first restriction enzyme recognition sequence are arranged in that order.

A recombinant vector containing a polymorphic nucleotide is produced using the recombinant vector of the present embodiment according to the method of the first embodiment. The recombinant vector containing a polymorphic nucleotide can be used to incorporate a polymorphic nucleotide into a nucleic acid, and a target sequence containing the polymorphic nucleotide can be prepared. Therefore, the present embodiment provides a recombinant vector for preparing a target sequence incorporating a polymorphic nucleotide. The recombinant vector of the present embodiment may be a recombinant vector for producing a recombinant vector containing a polymorphic nucleotide.

When the nucleic acid into which a polymorphic nucleotide is incorporated is a calibration reference material, the recombinant vector of the present embodiment may be a recombinant vector for producing a gene as a calibration reference material. When the calibration reference material is genomic DNA, the recombinant vector of the present embodiment preferably contains a reference sequence derived from the calibration reference material. The reference sequence may be an internal standard sequence.

### (Kit including recombinant vector for preparing target sequence incorporating polymorphic nucleotide, and nucleic acid sequences)

The third embodiment provides a kit including 1) a recombinant vector for preparing a target sequence incorporating a polymorphic nucleotide, having a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences, wherein the two first restriction enzyme recognition sequences are arranged such that cleavage sites for two first restriction enzymes are located on the sides opposite to the second restriction enzyme recognition sequence, and the first restriction enzyme recognition sequence is a Type IIS restriction enzyme recognition sequence, and the second restriction enzyme recognition sequence is a restriction enzyme recognition sequence different from the first restriction enzyme recognition sequence, 2) a first nucleic acid sequence to be inserted into a restriction enzyme recognition site, and containing a polymorphic nucleotide and its 5'-side and 3'-side flanking sequences, and 3) a second nucleic acid sequence to be inserted into a restriction enzyme recognition site and containing a complementary strand of the 2) first nucleic acid sequence.

In the kit of the present embodiment, the 2) first nucleic acid sequence and the 3) second nucleic acid sequence may be provided in a state in which they form a double strand. The double strand can be formed by annealing.

The 1) recombinant vector, the 2) first nucleic acid sequence, and the 3) second nucleic acid sequence provided as the kit of the present embodiment may be provided together with an arbitrary buffer, an enzyme or the like. For example, they may be provided together with a first restriction enzyme or a ligase, or may be provided together with a DNA or RNase inhibitor, a TE buffer or the like.

In the kit of the present embodiment, the 1) recombinant vector, the 2) first nucleic acid sequence, and the 3) second nucleic acid sequence may be provided in the same container or may be provided in separate containers. In addition, only one of the 1) recombinant vector, the 2) first nucleic acid sequence, and the 3) second nucleic acid sequence may be provided in a separate container. When the 1) recombinant vector, the 2) first nucleic acid sequence, and/or the 3) second nucleic acid sequence are provided in separate containers, these containers may be provided together in a single box or the like.

The kit of the present embodiment can produce a recombinant vector containing a polymorphic nucleotide by the method of the first embodiment, including reacting the 1) recombinant vector with a first restriction enzyme for cleavage, and ligating a double-stranded sequence composed of the 2) first nucleic acid sequence and the 3) second nucleic acid sequence. The recombinant vector containing a polymorphic nucleotide can be used to incorporate a polymorphic nucleotide into a nucleic acid, and a target sequence containing the polymorphic nucleotide can be prepared. Therefore, the present embodiment provides a kit for preparing a target sequence incorporating a polymorphic nucleotide. The kit of the present embodiment may be a kit for producing a recombinant vector containing a polymorphic nucleotide.

When the nucleic acid into which a polymorphic nucleotide is incorporated is a calibration reference material, the kit of the present embodiment may be a kit for producing a gene as a calibration reference material. When the calibration reference material is genomic DNA, the 1) recombinant vector in the kit of the present embodiment preferably contains a reference sequence derived from the calibration reference material. The reference sequence may be an internal standard sequence.

The present invention will be described below in detail with reference to examples, but the present invention is not limited to the examples.

### Examples

### <Restriction enzyme treatment and purification of cassette vector>

A KRAS-G12X/G13X cassette vector (Example 1: SEQ ID NO: 1), a KRAS-A59X/Q61X cassette vector (Example 2: SEQ ID NO: 2), a KRAS-K117X/A146X cassette vector (Example 3: SEQ ID NO: 3), a NRAS-G12X/G13X cassette vector (Example 4: SEQ ID NO: 4), a NRAS-A59X/Q61X cassette vector (Example 5: SEQ ID NO: 5), a NRAS-K117X/A146X cassette vector (Example 6: SEQ ID NO: 6), and a BRAF-V600X cassette vector (Example 7: SEQ ID NO: 7) were prepared by incorporating a sequence in which a partial gene sequence containing the exon region and the intron region was replaced with a 20-mer sequence having two BsmBl sites (5'-CGTCTC-3') flanking a Notl site (5'-GCGGCCGC-3') into a Tol2 vector. In the names of the cassette vectors, for example, "G12X/G13X" means that the cassette vector is for preparing a vector having a gene in which a sequence corresponding to glycine at position 12 and/or glycine at position 13 is substituted with a sequence corresponding to an arbitrary amino acid (X). The same applies to other cassette vectors. The prepared cassette vectors of SEQ ID NOs: 1 to 7 were subjected to a restriction enzyme treatment with BsmBI-v2 (New England BioLabs, Cat: R0739L) (55°C, 1 hour).

The digestion product was separated by electrophoresis on a 0.5% agarose/TAE gel and stained with an SYBR Gold staining reagent (Thermo Fisher Scientific, Cat: S11494) for 10 minutes to detect nucleic acids. A gel region including a target cleavage product was excised and purified using a QIAquick Gel Extraction Kit (QIAGEN, Cat: 28704).

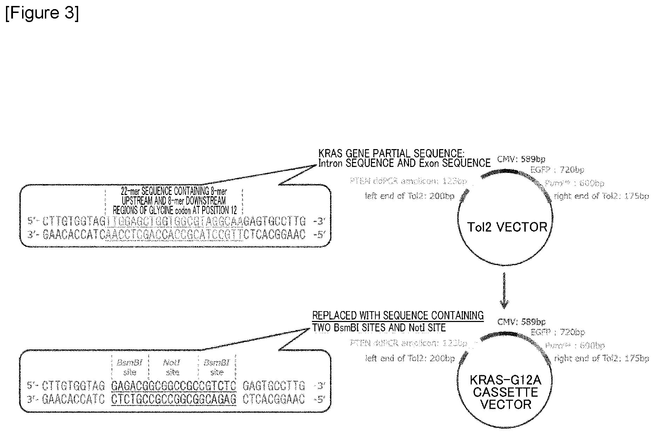

Here, the KRAS-G12X/G13X cassette vector (SEQ ID NO: 1) was a Tol2 vector having a KRAS gene partial sequence (sequence in which Exon2 was flanked by a 500-mer portion of Intron1 and a 500-mer portion of Intron2), which was a sequence in which a 22-mer sequence containing the 8-mer upstream and 8-mer downstream regions of the glycine codons at positions 12 and 13 was replaced with a 20-mer sequence having two BsmBI sites (5'-CGTCTC-3') flanking a Notl site (5'-GCGGCCGC-3'). Figure 3 shows an example of the KRAS-G12A cassette vector.

The KRAS-A59X/Q61X cassette vector (SEQ ID NO: 2) was a Tol2 vector having a KRAS gene partial sequence (sequence in which Exon3 was flanked by a 500-mer portion of Intron2 and a 500-mer portion of Intron3), which was a sequence in which a 21-mer sequence containing the 6-mer upstream and 6-mer downstream regions of the amino acid codons at positions 59 and 61 was replaced with a 20-mer sequence having two BsmBl sites (5'-CGTCTC-3') flanking a Notl site (5'-GCGGCCGC-3').

The KRAS-K117X/A146X cassette vector (SEQ ID NO: 3) was a Tol2 vector having a KRAS gene partial sequence (sequence in which Exon4 was flanked by a 500-mer portion of Intron3 and a 500-mer portion of Intron4), which was a sequence in which a 90-mer sequence containing the amino acid codons at positions 117 and 146 was replaced with a 20-mer sequence having two BsmBl sites (5'-CGTCTC-3') flanking a Notl site (5'-GCGGCCGC-3').

The NRAS-G12X/G13X cassette vector (SEQ ID NO: 4) was a Tol2 vector having an NRAS gene partial sequence (sequence in which Exon2 was flanked by a 500-mer portion of Intron1 and a 500-mer portion of Intron2), which was a sequence in which a 22-mer sequence containing the 8-mer upstream and 8-mer downstream regions of the glycine codons at positions 12 and 13 was replaced with a 20-mer sequence having two BsmBl sites (5'-CGTCTC-3') flanking a Notl site (5'-GCGGCCGC-3').

The NRAS-A59X/Q61X cassette vector (SEQ ID NO: 5) was a Tol2 vector having an NRAS gene partial sequence (sequence in which Exon3 was flanked by a 500-mer portion of Intron2 and a 500-mer portion of Intron3), which was a sequence in which a 21-mer sequence containing the 6-mer upstream and 6-mer downstream regions of the amino acid codons at positions 59 and 61 was replaced with a 20-mer sequence having two BsmBl sites (5'-CGTCTC-3') flanking a Notl site (5'-GCGGCCGC-3').

The NRAS-K117X/A146X cassette vector (SEQ ID NO: 6) was a Tol2 vector having an NRAS gene partial sequence (sequence in which Exon4 was flanked by a 500-mer portion of Intron3 and a 500-mer portion of Intron4), which was a sequence in which a 90-mer sequence containing the amino acid codons at positions 117 and 146 was replaced with a 20-mer sequence having two BsmBl sites (5'-CGTCTC-3') flanking a Notl site (5'-GCGGCCGC-3').

The BRAF-V600X cassette vector (SEQ ID NO: 7) was a Tol2 vector having a BRAF gene partial sequence (sequence in which Exon15 was flanked by a 500-mer portion of Intron14 and a 500-mer portion of Intron15), which was a sequence in which a 20-mer sequence containing the 8-mer upstream and the 9-mer downstream regions of the valine codon at position 600 was replaced with a 20-mer sequence having two BsmBI sites (5'-CGTCTC-3') flanking a Notl site (5'-GCGGCCGC-3').

### <Annealing reaction of DNA oligo containing each variant>

A TE buffer (1 mM EDTA, 10 mM Tris-HCl, pH 8.0) and 5 M NaCl were mixed at a ratio of 49:1, and to 10 µL of this mixed solution, 5 µL each of two types of 200 µM DNA oligos, whose sequences excluding the sticky ends to be formed were complementary to each other (Example 1: SEQ ID NOs: 8 to 31, Example 2: SEQ ID NOs: 32 to 51, Example 3: SEQ ID NOs: 52 to 61, Example 4: SEQ ID NOs: 62 to 85, Example 5: SEQ ID NOs: 86 to 117, Example 6: SEQ ID NOs: 118 to 127, and Example 7: SEQ ID NOs: 128 to 141) was added and mixed (a total volume of 20 µL). The prepared DNA oligo mixed solution was subjected to a thermal denaturation treatment at 95°C for 5 minutes, and cooled to room temperature at a rate of 1°C/min, and the two types of DNA oligos were annealed to form a double-stranded DNA oligo. The formed oligo was stored on ice or in a refrigerator set at 4°C until use.

### <Double-stranded DNA oligo ligation reaction>

The restriction enzyme-treated and gel-purified cassette vector and the annealed double-stranded DNA oligo were mixed at a molar ratio of 1:3, an equal volume of Ligation High Ver. 2 (TOYOBO, Cat: LGK-201) was added to the nucleic acid mixed solution, and the mixture was mixed and then reacted at 16°C for 30 minutes.

### <Transformation>

The ligation reaction solution was used to transform Competent high DH5α (TOYOBO, Cat: DNA-903). Transformed *E. coli* were seeded on an ampicillincontaining LB plate and cultured overnight under a condition of 37°C to form *E. coli* colonies.

### <Colony direct PCR>

An appropriate amount of an *E. coli* single colony formed by transformation was added to a 1×EmeraldAmp FastOCR Master Mix (Takara Bio, Cat: RR300A) containing 200 nM each of two types of primers of SEQ ID NOs: 141 and 142, a PCR reaction was performed for 30 cycles (one cycle: 98°C for 10 seconds, 55°C for 30 seconds, and 72°C for 75 seconds), the purified product was separated by electrophoresis on a 1.0% agarose/TAE gel and stained with an SYBR Gold staining reagent for 10 minutes to detect nucleic acids. For samples in which a PCR amplicon with a desired length was generated, insertion of the annealed double-stranded DNA oligo was confirmed by restriction enzyme digestion.

### <Confirmation of target DNA oligo insertion by restriction enzyme treatment of colony direct PCR product>

The colony direct PCR product obtained in the above <Colony direct PCR> section was subjected to a restriction enzyme treatment with Notl-HF (New England BioLabs, Cat: R3189S) (37°C, 1 hour). The digestion product was separated by electrophoresis on a 1.0% agarose/TAE gel and stained with an SYBR Gold staining reagent for 10 minutes to detect nucleic acids. When the PCR product was not digested with the restriction enzyme, the product was subjected to a direct sequencing method to determine the DNA sequence.

### <PCR product direct sequencing>

The concentration of the colony direct PCR product obtained in the above <Colony direct PCR> section was determined using a Qubit dsDNA BR Assay kit (Thermo Fisher Scientific, Cat: Q32853), 10 fg of the product treated with an ExoSAP-IT Exp reagent (applied buisystems, Cat: 75001.200.UL) was used as a template, and sequence analysis was performed at Eurofins Genomics using each of the two types of primers (SEQ ID NOs: 141 and 142) used in the colony direct PCR.

### <Large-scale purification and sequence confirmation of constructed vector>

Colonies confirmed to have the target sequence in the above <PCR product direct sequencing> section were cultured in an LB medium, and pDNA was purified from the obtained *E. coli* pellets using a Hispeed Plasmid Midi Kit (QIAGEN, Cat: 12643). Sequence analysis was performed at Eurofins Genomics using two types of primers of SEQ ID NOs: 141 and 142.

### Example 1

### <Result 1>

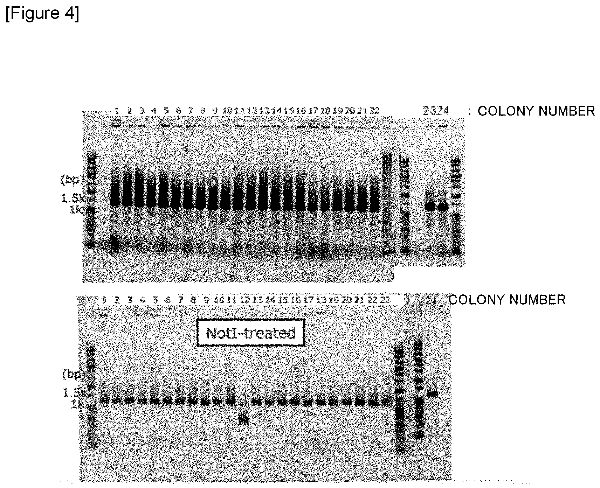

The restriction enzyme-treated and gel-purified cassette vector (KRAS-G12X/G13X cassette vector) prepared according to the experiment method and annealed double-stranded DNA oligos (each DNA oligo having a length of 28 mer) were ligated and transformed, and as a result, a plurality of colonies were formed. As a result of colony direct PCR, amplicons with a desired length were obtained for all of the 24 selected colonies, (Figure 4, the upper panel), and the results of the Notl treatment suggested that target annealed double-stranded DNA oligos were inserted in 23 colonies (Figure 4, the lower panel). For all colonies that were successfully sequence-analyzed, it was confirmed that the target sequence was incorporated. Since the recombination efficiency was extremely high, four single colonies each were selected and analyzed for the other G12X/G13X variants (a total of 11 variants), and as a result, the target recombination was confirmed in 41 colonies out of 44 colonies. As a result of sequence analysis using pDNA after large-scale purification of the constructed vector, it was confirmed again that the target sequence was incorporated.

### Example 2

### <Result 2>

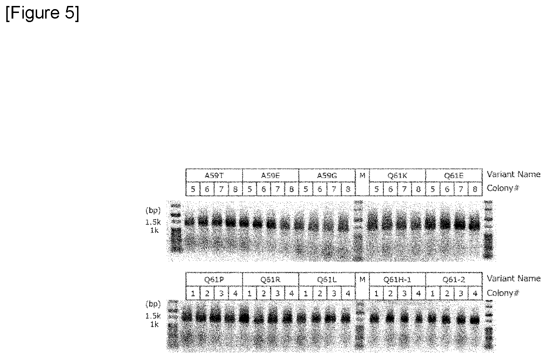

The restriction enzyme-treated and gel-purified cassette vector (KRAS-A59X/Q61X cassette vector) prepared according to the experiment method and annealed double-stranded DNA oligos (each DNA oligo having a length of 27 mer) were ligated and transformed, and as a result, a plurality of colonies were formed. As a result of colony direct PCR, amplicons with a desired length were obtained for all of the 4 selected colonies (Figure 5). As a result of direct sequence analysis of the obtained amplicons, the target recombination was confirmed in 38 colonies out of 40 colonies. As a result of sequence analysis using pDNA after large-scale purification of the constructed vector, it was confirmed again that the target sequence was incorporated.

### Example 3

### <Result 3>

The restriction enzyme-treated and gel-purified cassette vector (KRAS-K117X/A146X cassette vector) prepared according to the experiment method and annealed double-stranded DNA oligos (each DNA oligo having a length of 96 mer) were ligated and transformed, and as a result, a plurality of colonies were formed. 4 colonies each were selected, sequence analysis was performed, and as a result, the target recombination was confirmed in 16 colonies out of 20 colonies. Although the recombination success rate decreased due to the increase in the size of the incorporated double-stranded DNA oligo, the recombination efficiency was 80%. As a result of sequence analysis using pDNA after large-scale purification of the constructed vector, it was confirmed again that the target sequence was incorporated.

### Example 4

### <Result 4>

The restriction enzyme-treated and gel-purified cassette vector (NRAS-G12X/G13X cassette vector) prepared according to the experiment method and annealed double-stranded DNA oligos (each DNA oligo having a length of 28 mer) were ligated and transformed, and as a result, a plurality of colonies were formed. 4 colonies each were selected, sequence analysis was performed, and as a result, the target recombination was confirmed in 20 colonies out of 48 colonies. As a result of sequence analysis using pDNA after large-scale purification of the constructed vector, it was confirmed again that the target sequence was incorporated.

### Example 5

### <Result 5>

The restriction enzyme-treated and gel-purified cassette vector (NRAS-A59X/Q61X cassette vector) prepared according to the experiment method and annealed double-stranded DNA oligos (each DNA oligo having a length of 27 mer) were ligated and transformed, and as a result, a plurality of colonies were formed. 4 colonies each were selected, sequence analysis was performed, and as a result, the target recombination was confirmed in 60 colonies out of 64 colonies. As a result of sequence analysis using pDNA after large-scale purification of the constructed vector, it was confirmed again that the target sequence was incorporated.

### Example 6

### <Result 6>

The restriction enzyme-treated and gel-purified cassette vector (NRAS-K117X/A146X cassette vector) prepared according to the experiment method and annealed double-stranded DNA oligos (each DNA oligo having a length of 96 mer) were ligated and transformed, and as a result, a plurality of colonies were formed. 4 colonies each were selected, sequence analysis was performed, and as a result, the target recombination was confirmed in 8 colonies out of 20 colonies. Regarding K117N, sequence errors were observed in all analyzed colonies, and thus 12 colonies were additionally selected, sequence analysis was performed, and as a result, the target recombination was confirmed in 7 colonies. As a result of sequence analysis using pDNA after large-scale purification of the constructed vector, it was confirmed again that the target sequence was incorporated.

### Example 7

### <Result 7>

The restriction enzyme-treated and gel-purified cassette vector (BRAF-V600X cassette vector) prepared according to the experiment method and annealed double-stranded DNA oligos (each DNA oligo having a length of 26 mer) were ligated and transformed, and as a result, a plurality of colonies were formed. 4 colonies each were selected, sequence analysis was performed, and as a result, the target recombination was confirmed in 24 colonies out of 27 colonies. As a result of sequence analysis using pDNA after large-scale purification of the constructed vector, it was confirmed again that the target sequence was incorporated.

Table 1 shows the nucleic acid sequences used in this example. In the sequences of the cassette vectors of SEQ ID NOs: 1 to 7, only the cassette sequence portions are shown. For example, the KRAS-G12X/G13X cassette vector of SEQ ID NO: 1 was a KRAS gene partial sequence (sequence in which Exon2 was flanked by a 500-mer portion of Intron1 and a 500-mer portion of Intron2), which was a sequence in which a 22-mer sequence containing the 8-mer upstream and 8-mer downstream regions of the glycine codons at positions 12 and 13 was replaced with a 20-mer sequence having two BsmBI sites (5'-CGTCTC-3') flanking a Notl site (5'-GCGGCCGC-3'), as shown in Table 1. SEQ ID NOs: 2 to 6 are shown in Table 1 in the same manner as SEQ ID NO: 1.

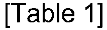

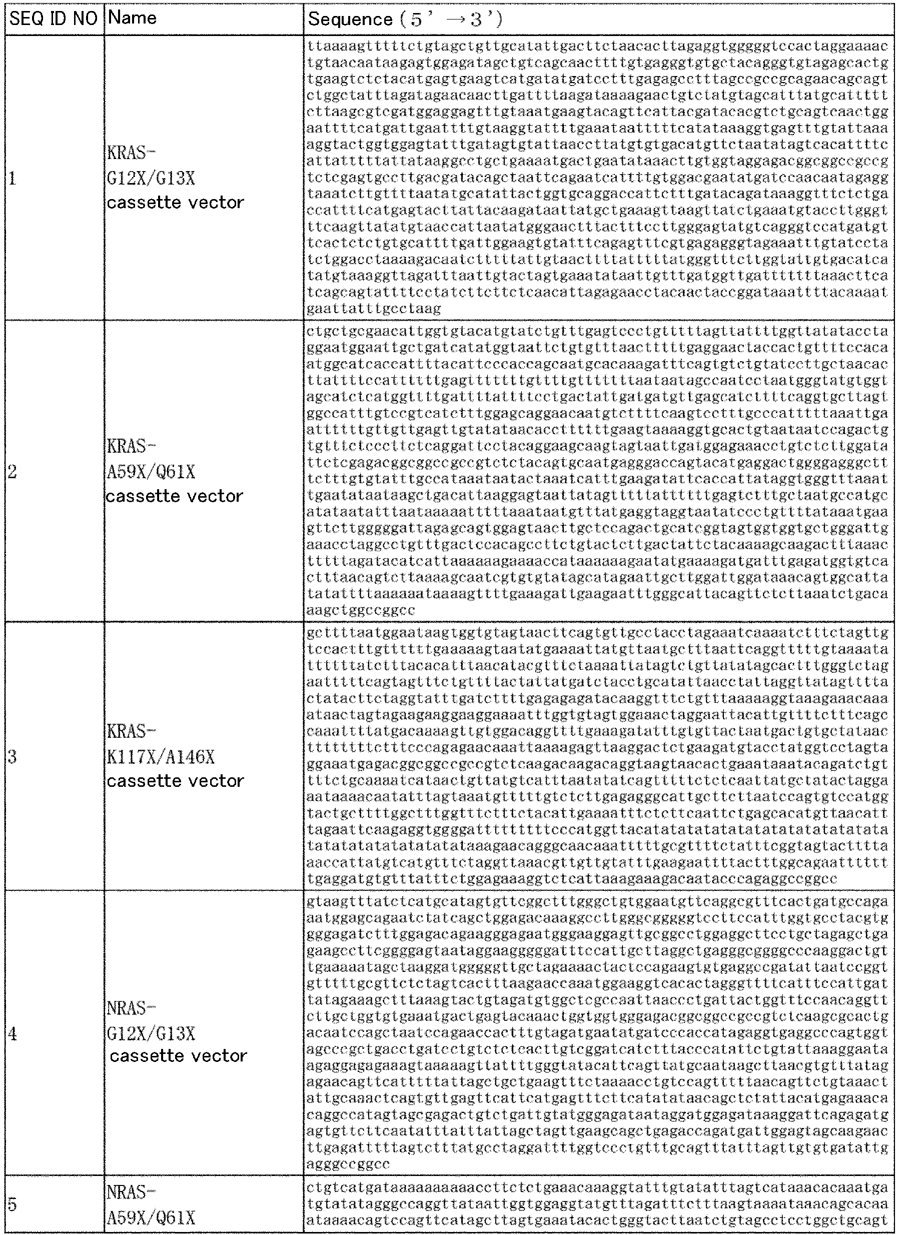

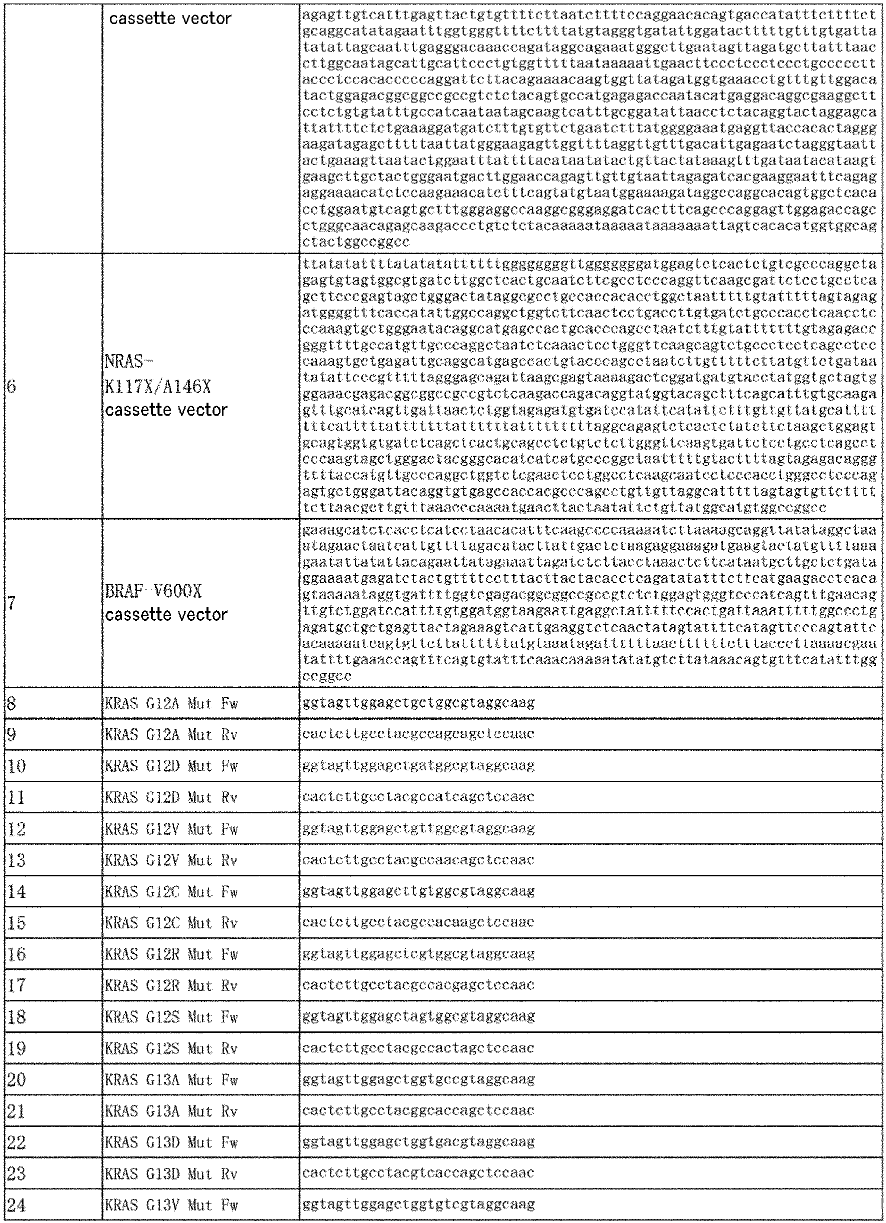

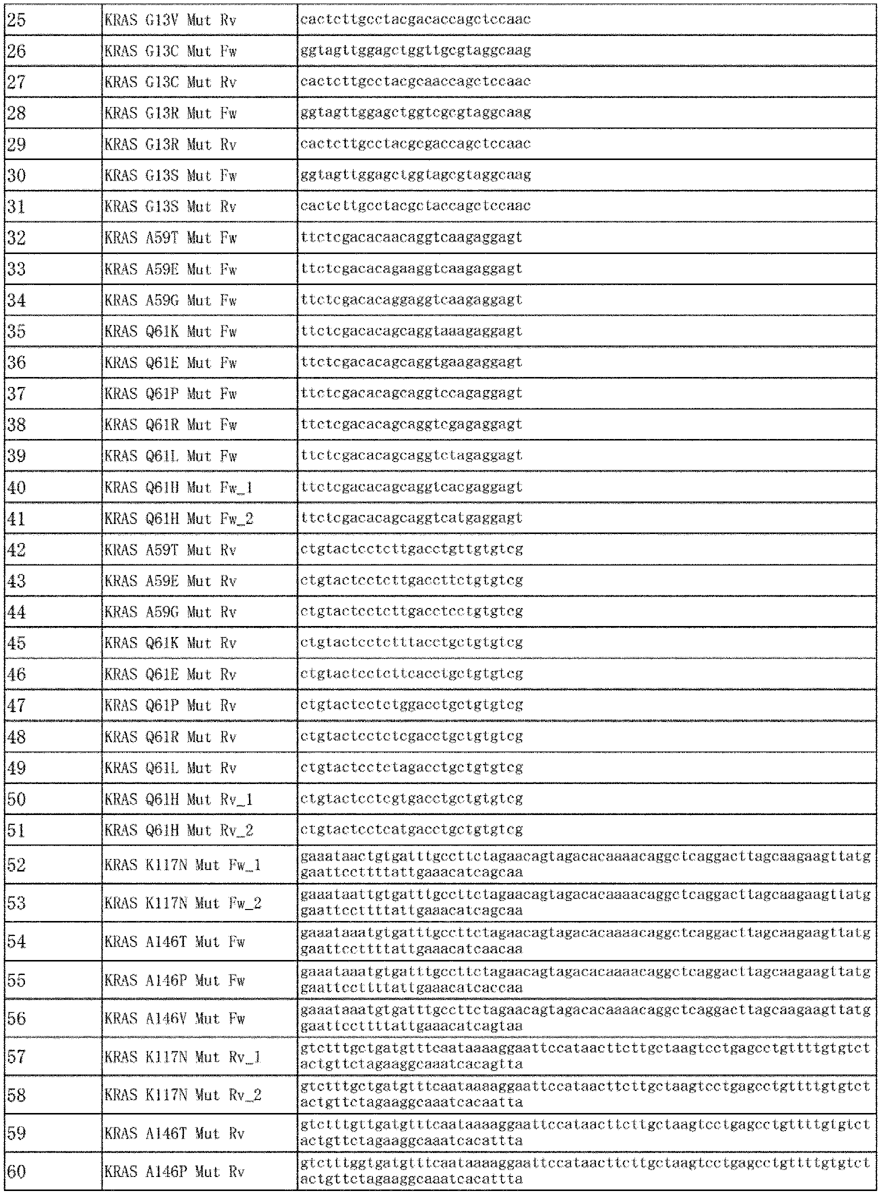

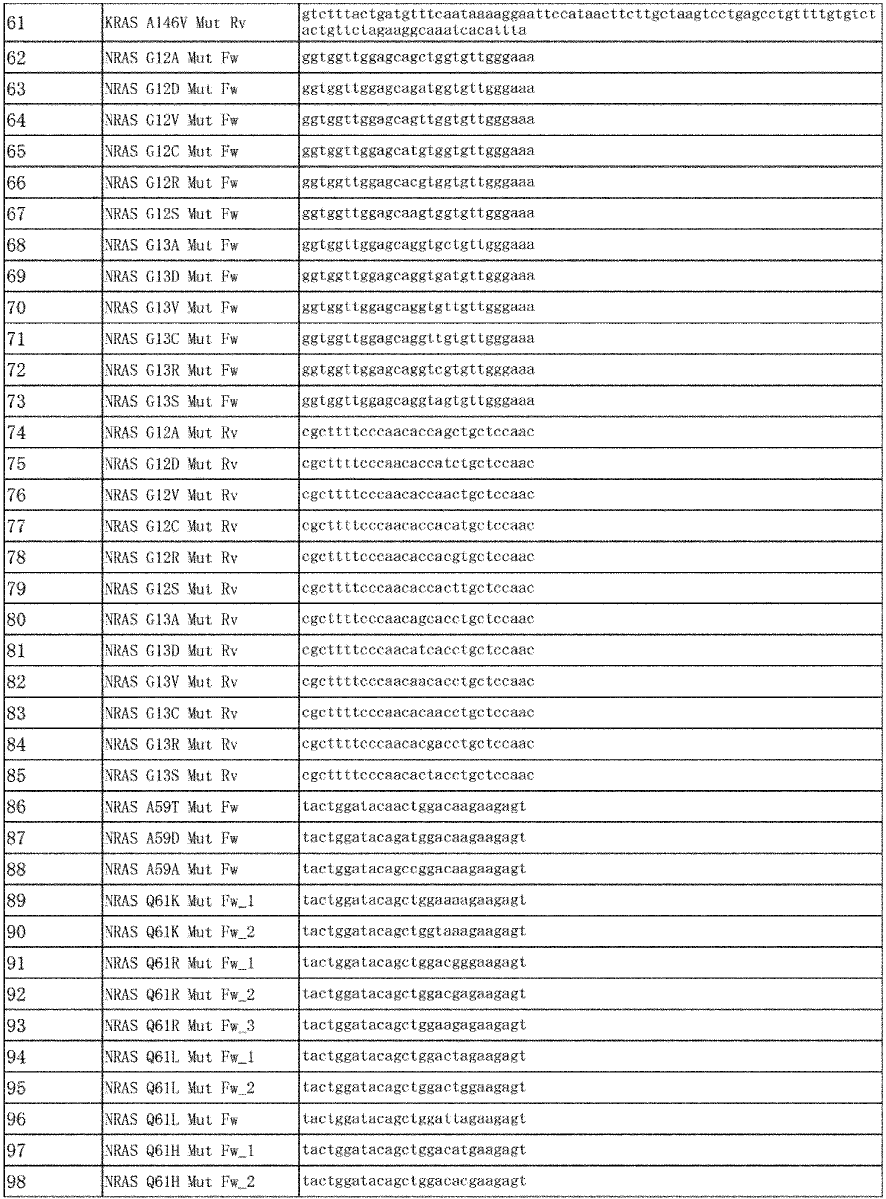

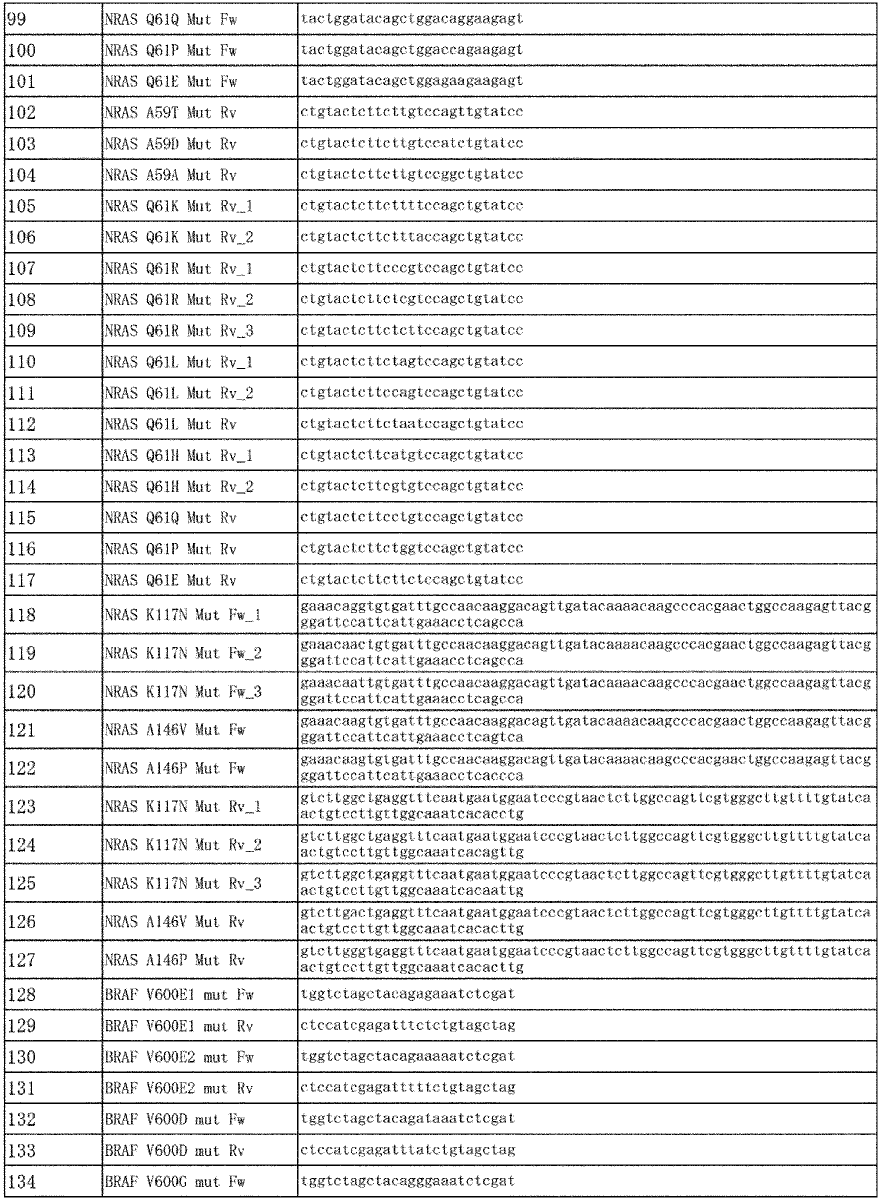

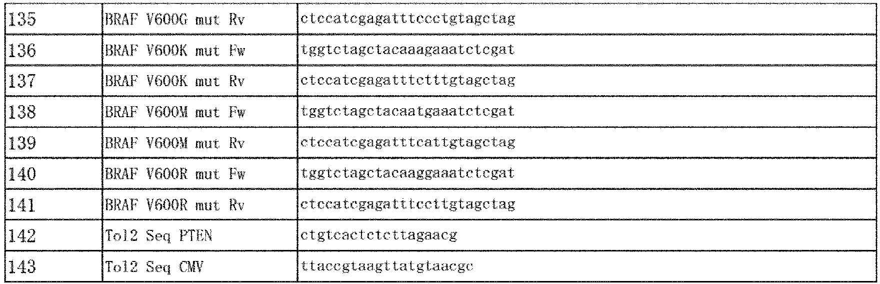

## Claims

1. A recombinant vector for preparing a target sequence incorporating a polymorphic nucleotide, having
a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences,
wherein the two first restriction enzyme recognition sequences are arranged such that cleavage sites for two first restriction enzymes are located on the sides opposite to the second restriction enzyme recognition sequence, and
the first restriction enzyme recognition sequence is a Type IIS restriction enzyme recognition sequence, and the second restriction enzyme recognition sequence is a restriction enzyme recognition sequence different from the first restriction enzyme recognition sequence.

2. The recombinant vector according to claim 1, wherein the two first restriction enzyme recognition sequences are arranged at least 3 nucleotides apart.

3. The recombinant vector according to claim 1 or 2, wherein the polymorphic nucleotide and its 5'-side and 3'-side flanking sequences have 10 to 200 nucleotide pairs.

4. The recombinant vector according to claim 1 or 2, which is a transposon vector.

5. The recombinant vector according to claim 1 or 2, wherein the Type IIS restriction enzyme is selected from the group consisting of Alwl, AlwXI, Alw26I, BbsI, Bbvl, Bbvll, Bcefl, Bccl, Bcgl, BciVI, BinI, Bmrl, Bpml, Bsal, BseRI, Bsgl, BsmAl, BsmBl, BspMI, BsrDI, BstF5I, Earl, Eco31I, Eco57I, Esp3I, Esp3I, Faul, Fokl, Gsul, Hgal, HinGUII, Hphl, Ksp632I, Mboll, Mmel, Mn1I, NgoVIII, PaqCl, Plel, Psrl, RleAI, Sapl, SfaNI, Taqll, Tth111II, Acul, Bsml, Bsrl, BsmFIBseMII, BspQl, and BtgZI.

6. The recombinant vector according to claim 1 or 2, wherein the target sequence is derived from genomic DNA.

7. The recombinant vector according to claim 6, wherein the recombinant vector further contains a reference sequence.

8. The recombinant vector according to claim 7, wherein the reference sequence is an internal standard sequence derived from genomic DNA.

9. A kit comprising:
1) a recombinant vector for preparing a target sequence incorporating a polymorphic nucleotide, having
a nucleic acid sequence in which a first restriction enzyme recognition sequence, a second restriction enzyme recognition sequence, and a first restriction enzyme recognition sequence are arranged in that order, corresponding to a region composed of a polymorphic nucleotide in a target sequence and its 5'-side and 3'-side flanking sequences together with their complementary sequences,
wherein the two first restriction enzyme recognition sequences are arranged such that cleavage sites for two first restriction enzymes are located on the sides opposite to the second restriction enzyme recognition sequence, and
the first restriction enzyme recognition sequence is a Type IIS restriction enzyme recognition sequence, and the second restriction enzyme recognition sequence is a restriction enzyme recognition sequence different from the first restriction enzyme recognition sequence,
2) a first nucleic acid sequence to be inserted into a restriction enzyme recognition site, and containing a polymorphic nucleotide and its 5'-side and 3'-side flanking sequences, and
3) a second nucleic acid sequence to be inserted into a restriction enzyme recognition site and containing a complementary strand of the 2) first nucleic acid sequence.

10. The kit according to claim 9, which is used to produce a gene as a calibration reference material.

11. The kit according to claim 10, wherein the gene as the calibration reference material is genomic DNA.

12. The kit according to claim 11, wherein the recombinant vector further contains an internal standard sequence derived from genomic DNA.

13. The kit according to any one of claims 9 to 12, wherein the 2) first nucleic acid sequence and the 3) second nucleic acid sequence form a double strand.

14. A method of producing a recombinant vector for preparing a target sequence containing a polymorphic nucleotide, comprising:
1) a step of cleaving a recombinant vector with a first restriction enzyme, wherein the recombinant vector has a nucleic acid sequence in which a first restriction enzyme recognition sequence, a second restriction enzyme recognition sequence, and a first restriction enzyme recognition sequence are arranged in that order,
the two first restriction enzyme recognition sequences are arranged such that cleavage sites for two first restriction enzymes are located on the sides opposite to the second restriction enzyme recognition sequence, and
the first restriction enzyme recognition sequence is a Type IIS restriction enzyme recognition sequence, and the second restriction enzyme recognition sequence is a restriction enzyme recognition sequence different from the first restriction enzyme recognition sequence; and
2) a step of ligating a double-stranded nucleic acid containing a region composed of a polymorphic nucleotide in a target sequence and its 5'-side and 3'-side flanking sequences together with their complementary sequences.

15. The method according to claim 14, further comprising a step of preparing a double-stranded nucleic acid by annealing.

16. The method according to claim 14 or 15, wherein the double-stranded nucleic acid has a sequence complementary to a cleavage sequence of the first restriction enzyme.

17. The method according to claim 14 or 15, further comprising a step of digesting with a second restriction enzyme.
